Europäisches Patentamt

European Patent Office

Office européen des brevets

(19)

(11) Veröffentlichungsnummer : **0 056 781**
**B1**

# (12) EUROPÄISCHE PATENTSCHRIFT

(45) Veröffentlichungstag der Patentschrift :
28.08.85

(21) Anmeldenummer : 82810001.6

(22) Anmeldetag : 04.01.82

(51) Int. Cl.⁴ : **A 61 K   9/50**

(54) **Verfahren zur Herstellung von liposomalen Arzneimitteln.**

(30) Priorität : 07.01.81 CH 53/81

(43) Veröffentlichungstag der Anmeldung :
28.07.82 Patentblatt 82/30

(45) Bekanntmachung des Hinweises auf die Patenterteilung : 28.08.85 Patentblatt 85/35

(84) Benannte Vertragsstaaten :
AT BE CH DE FR GB IT LI NL SE

(56) Entgegenhaltungen :
EP-A- 0 032 578
CHEMICAL ABSTRACTS, Band 95, Nr. 9, 31. August
1981, Nr. 76456k, Columbus, Ohio, USA R.A.
SCHWENDENER et al.: "n-Alkylglucosides as detergents for the preparation of highly homogeneous
bilayer liposomes of variable sizes (60-240 nm diam.)
applying defined rates of detergent removal by dialasis"
PHARMACEUTICAL ACTA HELVETICA 56, Nr. 4-5,
1981, Seiten 111-118, E. DOELKER et al.: "Formulation des comprimés à libération prolongée. III. Matrices lipidiques"

(73) Patentinhaber : Weder, Hans Georg, Prof. Dr.
Langmoosstrasse 52
CH-8135 Langnau am Albis (CH)

(72) Erfinder : Weder, Hans Georg, Prof. Dr.
Langmoosstrasse 52
CH-8135 Langnau Am Albis (CH)
Erfinder : Zumbühl, Otmar Norbert, Dr.
Hubelhuis
CH-6386 Wolfenschiessen (CH)
Erfinder : Schwendener, Reto Albert, Dr.
Surval
CH-7050 Arosa (CH)
Erfinder : Asanger, Maximilian, Dipl.-Pharm.
Eichbühlstrasse 66
CH-8004 Zurich (CH)

(74) Vertreter : Ryffel, Rolf
c/o Hepp Ryffel AG Bahnhofstrasse 58
CH-8001 Zürich (CH)

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

## Beschreibung

Die Erfindung bezieht sich auf ein Verfahren zur Herstellung von liposomalen Arzneimitteln, wobei in wässriger Phase aus wenigstens einer amphiphilen, bilayerbildenden Substanz und einem Lösungsvermittler, gegebenenfalls unter Zusatz von Arzneistoffen und/oder von pharmazeutischen Hilfsstoffen, Assoziate gebildet werden.

Bei den amphiphilen, bilayerbildenden Substanzen handelt es sich um Stoffe mit gleichzeitig polaren (hydrophilen) und apolaren (lipophilen) Eigenschaften, die in wässriger Phase Bilayer (Doppelschichten) bilden.

Liposomen sind kugelförmige Gebilde mit einem Durchmesser von 20 nm bis einigen μm. Sie bestehen aus mindestens einer Doppelschicht und umschliessen einen bestimmten, wässrigen Raum. Je nach der Anzahl der das wässrige Innenvolumen umschliessenden Doppelschichten spricht man von unilamellaren (eine Doppelschicht), oligolamellaren (einige wenige Doppelschichten) und multilamellaren (viele Doppelschichten) Liposomen.

Im Gegensatz zu unilamellaren Liposomen, bei denen ein ihrer Grösse entsprechendes, wässriges Innenvolumen eingeschlossen wird, werden bei oligo- bzw. multilamellaren Liposomen ihrer Anzahl Doppelschichten entsprechend mehrere voneinander getrennte wässrige Innenvolumina eingeschlossen.

Aufgrund ihrer physikalisch-chemischen Eigenschaften und ihrer Struktur können Liposomen als Träger für Arzneistoffe verwendet werden, wobei diese durch ihre hydrophilen und/oder lipophilen Eigenschaften entweder in den wässrigen Innenraum oder in die lipophilen Doppelschichten eingebaut oder an diese gebunden werden.

Pharmakodynamisch und/oder biologisch aktive Bilayerbildner bewirken, dass das Liposom selbst zum Arzneimittel wird. Je nach Applikationsart können die gebildeten, liposomalen Arzneimittel in entsprechende galenische Darreichungsformen übergeführt werden.

Liposomale Arzneimittel lassen sich zur gezielten Medikation für therapeutische und/oder diagnostische Zwecke sowie als Depotarzneiformen verwenden.

Ferner kann die Ueberführung des Arzneistoffes in ein liposomales Arzneimittel zur Verbesserung der Stabilität des Arzneistoffes und der resultierenden galenischen Darreichungsform führen.

Als Verfahren zur Herstellung von Liposomen und liposomalen Arzneimitteln sind bekannt :

1. Schütteln und/oder Beschallen von amphiphilen, bilayerbildenden Substanzen, gegebenenfalls unter Zusatz von Arzneistoffen und/oder pharmazeutischen Hilfsstoffen, in wässriger Phase.

2. Injektion von in organischen Lösungsmitteln, wie Aethanol oder Aether, gelösten amphiphilen bilayerbildenden Substanzen in ein wässriges Medium, wobei gegebenenfalls Arzneistoffe und/oder pharmazeutische Hilfsstoffe anwesend sein können.

3. Beschallen eines Systems, bestehend aus einer wässrigen und einer organischen Phase, die amphiphile, bilayerbildende Substanzen enthält, und Entfernen des organischen Lösungsmittels durch Verdampfen desselben, gegebenenfalls bei Anwesenheit von Arzneistoffen und/oder pharmazeutischen Hilfsstoffen.

4. Auflösen von amphiphilen, bilayerbildenden Substanzen in einem wässrigen Medium mit Hilfe von Lösungsvermittlern, wobei sich gemischte Mizellen bzw. Assoziate bilden, und anschliessendes Entziehen von Lösungsvermittler aus dem wässrigen Medium mittels Gelchromatographie oder Gleichgewichtsdialyse.

Diese Herstellungsverfahren sind jedoch alle mit meist mehreren der folgenden Nachteile behaftet : So führt jedes nicht schonende Herstellungsverfahren, wie z. B. die Ultrabeschallung, unweigerlich zu einer partiellen Degradation der amphiphilen, bilayerbildenden Substanzen sowie der in das Liposom einzuschliessenden Arzneistoffe, wie z. B. Proteine und Peptide. Bei der Verwendung von organischen Lösungsmitteln können diese nur noch mangelhaft aus dem gebildeten liposomalen Arzneimittel entfernt werden. Ebenso gelingt es mit derartigen Verfahren nicht, homogene liposomale Arzneimittel bezüglich des Dispersitätsgrades der darin enthaltenden Liposomen — es treten dabei Vesikelgrössen von 20 nm bis mehreren tausend nm auf — und/oder bezüglich der Anzahl Doppelschichten, die die innere, wässrige Phase der Liposomen umgrenzen, herzustellen. Eine exakte Dosierung der im liposomalen Arzneimittel vorliegenden Arzneistoffe ist daher kaum möglich.

Oftmals kann nur mit sehr verdünnten Dispersionen gearbeitet werden, so dass es nötig wird, will man zu einer für eine Medikation erforderlichen Konzentration des gebildeten liposomalen Arzneimittels gelangen, diese nachträglich mittels aufwendiger Verfahren, meist Ultrafiltration, aufzukonzentrieren.

Die bekannten Verfahren eignen sich auch nicht zur Herstellung von liposomalen Arzneimitteln im Produktionsmassstab, zumal meist noch zur unerlässlichen Erhöhung der Homogenität, sei es bezüglich des Dispersitätsgrades oder auch bezüglich der Anzahl der die wässrige Phase eines Liposoms umschliessenden Doppelschichten, weitere Trennverfahren, wie Ultrazentrifugation und/oder fraktionierte Filtration, angeschlossen werden müssen.

Das in-vivo-Verhalten der liposomalen Arzneimittel wird vom Dispersitätsgrad der darin enthaltenen Liposomen und von der Anzahl Doppelschichten, die die innere wässrige Phase der Liposomen umgrenzen, entscheidend beeinflusst. So erreicht man mit homogenen, unilamellaren Liposomen mit einem Durchmesser entsprechend der maximalen Porengrösse im sinusoidalen Kapillarbereich (ca.

100 nm) über längere Zeit hohe Blutspiegelwerte. Dagegen nehmen die Blutspiegelwerte von kleineren oder grösseren unilamellaren Liposomen signifikant ab und vor allem die Leberspiegelwerte entsprechend zu. Es besteht ferner die Möglichkeit, über die Grösse die Verteilung von homogenen unilamellaren Liposomen in bestimmten Organen, wie Milz, Niere und Lunge, zu steuern. Das in-vivo-Verhalten von polydispersen unilamellaren liposomalen Arzneimitteln ist nicht charakterisierbar, so dass eine kontrollierte Medikation nicht ermöglicht wird. Das in-vivo-Verhalten von multilamellaren, meist extrem polydispersen Liposomen ist dadurch gekennzeichnet, dass sie sich vor allem in Leber, Milz und Lunge anreichern, woraus äusserst niedrige Blutspiegel resultieren.

Der Erfindung lag daher die Aufgabe zugrunde, ein Verfahren zu entwickeln, das eine Degradation der amphiphilen, bilayerbildenden Substanzen und der Arzneistoffe weitgehend auszuschliessen gestattet und es ermöglicht, liposomale Arzneimittel — die frei von toxisch wirkenden Lösungsmitteln sind und eine extrem hohe Homogenität der Grössenverteilung sowie eine definierte Anzahl Doppelschichten aufweisen — möglichst rasch und gewünschtenfalls kontinuierlich in der für eine Medikation nötigen Menge mit erforderlichem Gehalt an Arzneistoff herzustellen.

In der nicht vorveröffentlichten EP-A-0 032 578, die aufgrund von Art. 54 Abs. 3 EPÜ zum Stand der Technik gehört, ist ein Verfahren der eingangs angegebenen Art beschrieben, mit dem Liposomen mit homogener Grösse hergestellt werden können. In diesem Verfahren wird aus der die Assoziate enthaltenden wässrigen Phase der Lösungsvermittler mittels Durchflussdialyse entfernt, wobei die Lösungsvermittlerkonzentration in der strömenden Dialysierflüssigkeit niedrig gehalten wird, damit der Konzentrationsgradient senkrecht zur semipermeablen Membran überall praktisch gleich ist.

Mit der vorliegenden Erfindung ist nun allgemein erkannt worden, dass sich Assoziate, denen Lösungsvermittler entzogen wird, zu Liposomen vereinigen, deren Grösse von der Geschwindigkeit des Entzugs von Lösungsvermittler bzw. von der Geschwindigkeit abhängt, mit der in der die Assoziate enthaltenden wässrigen Phase die Gleichgewichtsbedingungen im Sinne einer Erhöhung des molaren Verhältnisses von bilayerbildender Substanz zu Lösungsvermittler in den Assoziaten geändert werden.

Versuche, die im Nachstehenden noch beschrieben werden, haben gezeigt, dass man unilamellare Liposomen einer für ein gegebenes System minimalen Grösse erhalten kann, wenn man in der die Assoziate enthaltenden wässrigen Phase die Gleichgewichtsbedingungen für das molare Verhältnis zwischen bilayerbildender Substanz und Lösungsvermittler in den Assoziaten praktisch schlagartig ändert, z. B. durch plötzliches Verdünnen der wässrigen Phase (wodurch in derselben die Lösungsvermittlerkonzentration plötzlich erniedrigt wird). Bei langsamerer Aenderung der Gleichgewichtsbedingungen werden grössere Liposomen erhalten, deren Grösse dann praktisch konstant ist, wenn die genannten Gleichgewichtsbedingungen mit einer zeitlich und örtlich (bei allen Assoziaten in der wässrigen Phase) praktisch konstanten Geschwindigkeit geändert werden. Wenn die Aenderung der Gleichgewichtsbedingungen mit einer konstanten Geschwindigkeit erfolgt, die niedriger ist als eine erste empirisch bestimmbare obere Grenzgeschwindigkeit, bei der sich unilamellare Liposomen einer maximalen Grösse bilden, dann entstehen Liposomen mit einer definierten mehrzahl von Doppelschichten, wobei die Grössenverteilung noch homogen ist. Eine zweite, im Einzelfall ebenfalls durch Versuch leicht feststellbare untere Grenzgeschwindigkeit der Aenderung der Gleichgewichtsbedingungen darf jedoch nicht unterschritten werden, weil sich unterhalb dieser zweiten Grenzgeschwindigkeit Liposomen mit nicht mehr definierter Doppelschichtenzahl und heterogener Grössenverteilung bilden.

Nachstehend werden verschiedene mögliche Ausführungsformen der Erfindung erörtert.

So kann man zunächst zur Herstellung von Assoziaten amphiphile, bilayerbildende Substanzen, gegebenenfalls unter Zusatz von Arzneistoffen und/oder pharmazeutischen Hilfsstoffen, in einen festen Zustand mit möglichst grosser Oberfläche überführen. Dies kann in bekannter Weise mit den oben erwähnten, in organischen Lösungsmitteln oder deren Gemischen gelösten Substanzen dadurch erreicht werden, dass die Lösungsmittel verdampft werden, so dass beispielsweise an der Glaswand eines Rundkolbens ein entsprechend dünner Film des Substanzgemisches entsteht, oder dadurch, dass die Lösungsmittel durch Lyophilisation (Gefriertrocknung) entfernt werden.

Die zur Bildung der Assoziate in wässriger Lösung erforderlichen Lösungsvermittler können entweder der organischen Phase direkt zugefügt oder in der anschliessend dem hochdispersen Substanzgemisch zuzufügenden wässrigen Phase gelöst werden. Falls erforderlich, können die Lösungsvermittler sowohl im organischen Lösungsmittelgemisch als auch in der wässrigen Phase enthalten sein.

Es wird dann vorzugsweise die Menge an wässriger Phase mit definierter Ionenstärke (vorzugsweise zwischen 0 und etwa 0,3) und definiertem pH-Wert (vorzugsweise zwischen etwa 1 und 10) hinzugefügt, die ausreicht, um die darin gebildeten Assoziate gerade noch in Lösung zu halten. Der pH-Wert wird zweckmässig durch Zugabe eines Puffergemischs aufrechterhalten. Die Konzentration dieses Puffers ist nicht kritisch, die gewählte Ionenstärke kann durch Zusatz von grösseren oder geringeren Mengen neutraler Salze, z. B. NaCl, eingehalten werden. Gut in Wasser lösliche Arzneistoffe und/oder pharmazeutische Hilfsstoffe können auch direkt in der zuzufügenden wässrigen Phase enthalten sein.

Die Assoziat-Lösung kann etwa 1 bis 150, vorzugsweise 10 bis 100 mg/ml Bilayer-Bildner und etwa 1 bis 200, vorzugsweise 5 bis 100 mg/ml Lösungsvermittler enthalten. Das molare Verhältnis von Bilayerbildner zu Lösungsvermittler liegt zweckmässig zwischen etwa 0,1 und 2.

In der die Assoziate enthaltenden wässrigen Phase müssen nun die Gleichgewichtsbedingungen für das molare Verhältnis von bilayerbildender Substanz zu Lösungsvermittler in den Assoziaten im Sinne einer Erhöhung dieses Verhältnisses geändert werden. Zu diesem Zweck kann man beispielsweise

A. die totale Lösungsvermittlerkonzentration in der wässrigen Phase durch Verdünnung derselben erniedrigen oder

B. durch chemische und/oder physikalisch-chemische Reaktionen des Lösungsvermittlers in der wässrigen Phase dem Assoziat Lösungsvermittler entziehen oder

C. durch Gegenstromdialyse der wässrigen Phase die totale Lösungsvermittlerkonzentration erniedrigen oder

D. die totale Bilayerbildnerkonzentration in der wässrigen Phase durch Zusatz von Bilayerbildner erhöhen.

Die « totale Lösungsvermittlerkonzentration » bezeichnet hier den auf wässrige Phase bezogenen Gesamtgehalt an Lösungsvermittler in der wässrigen Phase und in den Assoziaten.

Für die Herstellung von liposomalen Arzneimitteln mittels Verdünnung (A) kann man die die Assoziate enthaltende wässrige Phase mit einer zusätzlichen Menge an wässriger Phase derart verdünnen, dass die Lösungsvermittlerkonzentration auf vorzugsweise weniger als 25 % der Ausgangskonzentration herabgesetzt wird (wobei die Ausgangskonzentration vorzugsweise nicht oder nicht wesentlich höher ist als die zum Solubilisieren der gesamten Bilayerbildnermenge erforderliche). Dadurch wird den Assoziaten Lösungsvermittler entzogen, was eine Umstrukturierung der bilayerbildenden Substanzen zu Doppelschichten bewirkt, woraus Liposomen bzw. liposomale Arzneimittel entstehen.

Die zuzusetzende wässrige Phase besteht vorzugsweise ebenfalls aus Pufferlösung unter Zusatz von Salzen, wie Natriumchlorid. Der pH-Wert der wässrigen Phase liegt im allgemeinen zwischen etwa 1 und etwa 10, vorzugsweise zwischen 5 und 8, die Ionenstärke zwischen 0 und etwa 0,3.

Sind die Lösungsvermittler im vorstehend beschriebenen hochdispersen Substanzgemisch in genügender Menge vorhanden oder werden sie diesem in der erforderlichen Menge zugesetzt und erfolgt bei Zusatz von wässriger Phase die Bildung der Assoziate augenblicklich, so kann die Bildung der Liposomen bzw. der liposomalen Arzneimittel durch einfaches Zufügen einer ausreichenden Menge, vorzugsweise von etwa 2 bis 25 Volumenteilen, an wässriger Phase zu einem Volumenteil des hochdispersen Substanzgemischs unmittelbar erreicht werden. Ein anschliessendes Konzentrieren kann erforderlich sein.

Die Grösse der im liposomalen Arzneimittel enthaltenen Liposomen kann z. B. durch die Wahl der absoluten Konzentration an amphiphilen, bilayerbildenden Substanzen und/oder durch Variation des molaren Verhältnisses zwischen Bilayerbildner und Lösungsvermittler und/oder durch die Wahl des oder der Lösungsvermittler bzw. ihrer Mischungsverhältnisse gesteuert werden. Entsprechende Ergebnisse sind in der Tabelle im Beispiel 1 zusammengefasst. Ferner ist die Grösse der Liposomen von der Verdünnungszeit abhängig ; diese liegt zweckmässig zwischen etwa 10 msec (praktisch unmessbar kurz) und etwa drei Stunden.

Bei der Herstellung der liposomalen Arzneimittel ist die Temperatur vorzugsweise derart zu wählen, dass sich die Assoziate in wässriger Phase möglichst rasch bilden und dass deren Stabilität bis zum anschliessenden Verdünnungsschritt gewährleistet ist. Letzteren führt man zweckmässig bei einer Temperatur durch, die etwa 5 bis 25 °C höher ist als die entsprechenden Uebergangstemperaturen der amphiphilen, bilayerbildenden Substanzen. Die Uebergangstemperatur einer bilayerbildenden Substanz ist die Temperatur des Phasenübergangs kristallin-flüssigkristallin der in wässriger Phase gebildeten Doppelschichten. Allgemein arbeitet man bei Temperaturen zwischen etwa 0 und etwa 100°, vorzugsweise zwischen 10 und 70 °C.

Die Herstellung von liposomalen Arzneimitteln durch chemische und/oder physikalisch-chemische Inaktivierung (B) des Lösungsvermittlers in der die Assoziate enthaltenden wässrigen Phase kann wie folgt erfolgen :

a) Plötzliche Aenderung der Temperatur (Temperatursprung) in der wässrigen Phase, wobei die Löslichkeit der Lösungsvermittler stark herabgesetzt wird und/oder die Interaktionskräfte zwischen Lösungsvermittler und Bilayerbildnern beeinflusst werden und somit Lösungsvermittler den Assoziaten entzogen wird. Der Temperatursprung führt in der Regel von einer Temperatur, die etwa 5 bis 25 °C über der Uebergangstemperatur liegt, zu einer Temperatur, die so tief wie möglich darunter liegt, insbesondere von Temeraturen zwischen etwa 30 und 100 °C zu Temperaturen zwischen etwa 0 und 20 °C.

b) Zusatz geeigneter Adsorbentien, z. B. Aktivkohle, Kieselgele, fein verteilte Kieselsäure, die mit der wässrigen Phase in Kontakt treten. Dadurch können die in der wässrigen Phase und in den Assoziaten vorhandenen Lösungsvermittler entzogen werden.

c) Plötzliche Aenderung des pH-Wertes (pH-Sprung) der wässrigen Phase, wobei die Lösungsvermittler z. B. durch Ausfällung der wässrigen Phase entzogen werden, durch Zufügen von Säure (z. B. Salzsäure), Base (z. B. Natronlauge) oder sauren bzw. basischen Ionenaustauschern. Die Einzelbedingungen sind im wesentlichen vom $pK_a$-Wert des Lösungsvermittlers abhängig.

d) Zufügen einer weiteren Substanz zur wässrigen Phase, die zur Komplexierung und/oder Ausfällung der darin enthaltenen Lösungsvermittler führt. Dies kann z. B. dadurch erfolgen, dass kationische bzw. anionische Lösungsvermittler durch strukturell geeignete anionische bzw. kationische Substanzen ausgefällt werden (ein quaternäres Ammoniumsalz fällt nahezu alle anionenaktiven Lösungsvermittler).

4

Die Herstellung von liposomalen Arzneimitteln durch Gegenstromdialyse (C) erfordert eine semipermeable Membran, die die amphiphilen, bilayerbildenden Substanzen in der zu dialysierenden, wässrigen Phase möglichst vollständig zurückhält, jedoch für die Lösungsvermittler eine hohe Permeabilität aufweist. Ferner soll sie so beschaffen sein, dass sie die in der zu dialysierenden, wässrigen Phase enthaltenen Arzneistoffe und/oder pharmazeutischen Hilfsstoffe im gewünschten Masse zurückhält. Bei diesen semipermeablen Membranen handelt es sich vorzugsweise um Membranen aus Cellulose, hydratisierter Cellulose, regenerierter Cellulose (Zellglas) und Cellulosederivaten wie Acetylcellulose, deren Dicke im μm-Bereich liegt und die eine molekulare Ausschlussgrenze im Bereiche von 1 000 bis 10 000 Molekulargewicht aufweisen. Ferner geeignet sind Membranen aus Polyamiden (z. B. Nylon), Polyenen, wie Polyethylen oder Polypropylen, Polyestern, Polyvinylchlorid, Polytetrafluorethylen und Polycarbonaten.

Die bevorzugte Membrandicke liegt zwischen etwa 5 und etwa 20 μm.

Wenn die zu dialysierende, wässrige Phase und die Dialysierflüssigkeit, voneinander durch die oben beschriebene Membran getrennt, im Gegenstrom auf einem erzwungenen Weg in möglichst geringer Schichdicke, vorzugsweise 0,1 bis 1 mm, fliessen, wird der Lösungsvermittler sehr rasch und wenn gewünscht auch vollständig (< 1 % der Ausgangskonzentration) der zu dialysierenden, wässrigen Phase entzogen, wobei sich das liposomale Arzneimittel bildet.

Vorzugsweise wird der Fluss der Dialysierflüssigkeit derart gesteuert, dass die Konzentrationen der zu dialysierenden Substanzen in Bewegungsrichtung der Dialysierflüssigkeit kontinuierlich zunehmen. Dabei kann die Strömungsgeschwindigkeit der Dialysierflüssigkeit zweckmässig derart gewählt werden, dass das über der semipermeablen Membran gebildete Konzentrationsgefälle der zu dialysierenden Substanzen möglichst gross ist.

Mit diesem Gegenstromdialyseverfahren kann die Voraussetzung erfüllt werden, dass der Kontakt zwischen der zu dialysierenden, wässrigen Phase und der Dialysierflüssigkeit über die semipermeable Membran so lange aufrechterhalten wird, bis die Bildung der liposomalen Arzneimittel abgeschlossen und die darin enthaltenen Lösungsvermittler auf die gewünschte Konzentration reduziert worden sind. In der Regel ist das nach etwa 1 bis 120 Minuten der Fall.

Dieses Durchflussverfahren weist im Vergleich zu den bis jetzt bekannten Verfahren den bedeutenden Vorteil auf, dass durch eine mit hoher und (örtlich und zeitlich) praktisch konstanter Geschwindigkeit erfolgende Herabsetzung der Lösungsvermittlerkonzentration in der wässrigen Phase Liposomen bzw. liposomale Arzneimittel, die extreme Homogenität in Bezug auf die Grössenverteilung und die Anzahl der die innere, wässrige Phase umschliessenden Doppelschichten aufweisen, extrem rasch und kontinuierlich hergestellt werden können, wobei jede Verdünnung des resultierenden Arzneimittels vermieden werden und der Herstellungsprozess laufend überwacht werden kann. Durch geeignete Wahl des Verhältnisses der Strömungsgeschwindigkeit der zu dialysierenden, wässrigen Phase und der Dialysierflüssigkeit, vorzugsweise etwa 1 : 6 bis 1 : 10, kann die Dialysiergeschwindigkeit der Lösungsvermittler und damit die Grösse der gebildeten Liposomen gesteuert werden, indem mit im Verhältnis zur Strömungsgeschwindigkeit der zu dialysierenden, wässrigen Phase steigender Strömungsgeschwindigkeit in der Dialysierflüssigkeit die Grösse der sich bildenden Liposomen abnimmt. Ferner kann die Grösse der Liposomen wie schon beschrieben durch die Wahl der Konzentrationen, der molaren Verhältnisse und der Art des Lösungsvermittlers in ähnlicher Weise wie bei der Verdünnungsmethode gesteuert werden.

In der Europäischen Patentanmeldung 0 032 578 ist ein Verfahren zur Herstellung von Bilayer-Vesikeln mittels Durchflussdialyse beschrieben. Dieses Verfahren unterscheidet sich von dem hier vorliegenden Gegenstromdialyse-Verfahren dadurch, dass dort eine Phase stationär ist, während sich hier beide Phasen gegeneinander bewegen. Das ältere Verfahren kann daher im Gegensatz zu dem vorliegenden nicht kontinuierlich betrieben werden.

Die Herstellung von liposomalen Arzneimitteln durch Erhöhung der Bilayerbildnerkonzentration (D) in der wässrigen Phase, die die Assoziate und gegebenenfalls Arzneistoffe und/oder pharmazeutische Hilfsstoffe enthält, kann wie folgt durchgeführt werden : Ein Film oder ein Lyophilisat aus bilayerbildenden Substanzen kann wie im Vorstehenden beschrieben, gegebenenfalls mit den angegebenen Zusätzen, hergestellt werden. Anschliessend kann eine wässrige Phase zugegeben werden, die bereits Assoziate aus Bilayerbildner und Lösungsvermittler, ferner nicht in Assoziaten gebundene Lösungsvermittler, Arzneistoffe und gegebenenfalls pharmazeutische Hilfsstoffe enthält. Durch anschliessendes, vorsichtiges Schütteln können dann mittels der in der wässrigen Phase noch freien Lösungsvermittler die bilayerbildenden Substanzen des Lipidfilmes bzw. des Lyophilisates in Assoziate übergeführt werden, wobei sich im Vergleich zu der ursprünglich zugesetzten wässrigen Phase ein neues molares Verhältnis zwischen Bilayerbildnern und Lösungsvermittlern zugunsten der Bilayerbildner einstellt und somit spontan liposomale Arzneimittel entstehen.

Eine weitere Möglichkeit besteht darin, dass amphiphile, bilayerbildende Substanzen in zwei getrennten Gefässen vorab wie beschrieben in Assoziate übergeführt werden und sich durch Mischen beider Assoziatlösungen ein neues Gleichgewicht zwischen Bilayerbildnern und Lösungsvermittlern zugunsten der Bilayerbildner einstellt. Werden dabei zur Herstellung der beiden Assoziatlösungen dieselben Lösungsvermittler verwendet, jedoch in unterschiedlicher Konzentration, dann stellt sich nach Mischen der beiden Assoziatlösungen gezwungenermassen ein neues Verhältnis zwischen Bilayerbildner und Lösungsvermittler ein, was wiederum bei geeigneter Wahl der Lösungsvermittler zur Bildung von liposomalen Arzneimitteln führt. Bei Verwendung von unterschiedlichen, strukturell geeigneten Lösungsvermittlern zur Herstellung der beiden Assoziatlösungen ist es beim Mischen der beiden Lösungen möglich, durch chemische und/oder physilakisch-chemische Reaktionen der Lösungsvermittler untereinander diese vollständig oder partiell zu inaktivieren, was spontan zur Bildung von liposomalen Arzneimitteln führt. Werden die amphiphilen, bilayerbildenden Substanzen zur Herstellung der beiden Assoziatlösungen jeweils mit Lösungsvermittlern unterschiedlicher solubilisierender Eigenschaften versetzt, so dass beim Mischen beider Assoziatlösungen die eingesetzten Lösungsvermittler nur die Bilayerbildner der ursprünglichen Assoziatlösungen zu solubilisieren vermögen, entsteht durch Transfer der bilayerbildenden Substanzen zwischen den beiden Assoziaten in diesen ein neues molares Verhältnis zwischen Bilayerbildnern und Lösungsvermittlern zugunsten der Bilayerbildner, was zur Bildung von liposomalen Arzneimitteln führt.

Für die Herstellung von liposomalen Arzneimitteln, wie vorstehend beschrieben, kommen amphiphile, bilayerbildende Substanzen natürlicher oder synthetischer Herkunft in Betracht, d. h. Stoffe mit gleichzeitig polaren (hydrophilen) und apolaren (lipophilen) Eigenschaften.

Im einzelnen eignen sich als Bilayerbildner insbesondere Phospholipide, beispielsweise Phosphoglyceride (Diester, Monoester, Diether, Monoether, wobei die Ester- und Ethergruppen vorzugsweise jeweils etwa 8 bis 24 C-Atome besitzen), wie Lecithine (Phospatidylcholine), Kephaline (Phosphatidylethanolamine, Phosphatidylserine), Inositphosphatide, Phosphatidylsäuren, Phosphatidylglycerin, Cardiolipin ; Sphingolipide, z. B. Sphingomyelin ; Glycolipide, z. B. Cerebroside, Ganglioside ; insbesondere auch synthetische Glycolipide wie 6-(1-Thio-1-desoxy-β-D-galactopyranosyl)-1-(5-cholesten-3β-yloxy)-hexan.

Ferner kommen die folgenden Stoffgruppen in Betracht, die meist mit anderen amphiphilen, bilayerbildenden Substanzen in wässriger Phase Doppelschichten bilden : Fettsäuren mit vorzugsweise 8 bis 24 C-Atomen sowie deren Ester, Salze und Amide ; Alkylether mit vorzugsweise 8 bis 24 C-Atomen : Alkyletherderivate mit vorzugsweise 8 bis 24 C-Atomen, z. B. 1,3-Propandiol-phospholipide ; höhere Alkylamine mit vorzugsweise 8 bis 24 C-Atomen, z. B. Steraylamin ; Fettalkohole mit vorzugsweise 8 bis 24 C-Atomen (z. B. Stearylalkohol) sowie deren Ester (z. B. Dicetylphosphat), höhere Alkylthiole mit vorzugsweise 8 bis 24 C-Atomen. Ferner kommen Gemische dieser Substanzen in Betracht. Allgemein können die Alkylketten der genannten Stoffe unverzweigt, verzweigt und/oder cyclisch sein.

Polymerisierbare Derivate von Substanzen sämtlicher oben erwähnter Stoffgruppen, wie z. B. Diacetylenphospholipide, sind ebenfalls als Bilayerbildner geeignet, wobei diese in der Doppelschicht nach Bildung der Liposomen durch bekannte Methoden vernetzt werden können.

Als Lösungsvermittler sind Substanzen aus den folgenden Gruppen mit Vorteil einzusetzen :

1. Cholsäure, deren Salze und Derivate, wie Desoxycholsäure, Taurocholsäure, Chenodesoxycholsäure, Lithocholsäure und Glycocholsäure, wobei vorzugsweise deren Natriumsalze eingesetzt werden.

2. Monomere oder oligomere Zuckerderivate, bekannt als Glycoside, mit lipophiler Seitenkette, z. B. 1-0-n-Octyl-β-D-gluco-pyranosid oder die entsprechenden Hexyl-, Heptyl- bzw. Nonyl-Analoga, wobei sich auch Mischungen solcher Glycoside sehr gut eignen.

3. Ionogene Stoffe, davon als anionenaktive Lösungsvermittler Na- und K-Salze von Fettsäuren mit vorzugsweise 8 bis 24 C-Atomen, Aminseifen (z. B. Triethanolaminstearat), Salze von Schwefelsäure- und Sulfonsäureestern höherer Fettalkohole mit vorzugsweise 8 bis 24 C-Atomen (z. B. Natriumlaurylsulfat, Docusat-Natriumsalz U.S.P. 20, Natriumlaurylsulfonat). Als kationenaktive Lösungsvermittler kommen z. B. quaternäre Ammoniumverbindungen in Frage.

4. Weitere, nicht ionogene Lösungsvermittler, z. B. Partialfettsäureester mehrwertiger Alkohole (Glycerin monostearat, Pentaerythritmonostearat, Partialfettsäureester des Sorbitans (Span®, Crill®), Partialfettsäureester des Polyoxiethylensorbitans (Tween®), Umsetzungsprodukte von Ricinusöl oder hydriertem Ricinusöl mit Ethylenoxid (z. B. Cremophor® EL), ethoxylierte gesättigte Fettalkohole (z. B. Cremophor® A und O, Brij®), Fettsäure-polyethylenglykol-ester (z. B. Cremophor® AP, Myrj®), Polyetheralkohole (z. B. Pluronic®), sowie Polyethylenglykole (z. B. Triton®).

Detergens und Bilayerbildner bilden mit Wasser ein ternäres System, hier als gemischte Mizelle bezeichnet. Die kolloidale Lösung der gemischten Mizelle, im weiteren Mizell-Lösung genannt, kann zusätzlich Elektrolyte (vorwiegend physiologisch unbedenkliche anorganische Salze wie Natriumchlorid, Natriummono- und -dihydrogenphosphat, Kaliummono- und -dihydrogenphosphat), Sorptionsvermittler (wie organische Lösungsmittel, Fettalkohole und Fettsäureester), Hilfsstoffe (wie Stabilisierungs- und Konservierungsmittel), Peptide, Proteine, Nukleinsäuren, Lipide, Antigene und Antikörper sowie Wirkstoffe mit biologischen und pharmakodynamischen Eigenschaften enthalten. Als geeignete Wirkstoffe seien beispielsweise Arzneimittelwirkstoffe und deren Derivate genannt : Steroide, z. B. Sterine, wie Cholesterin, Sitosterin ; Œstrogene, wie Estron, Estradiol und dessen Ester, Ethinylestradiol ; Gestagene, wie Norethisteronacetat, Chlormadinoacetat ; Corticoide, wie Hydrocortison, Cortison, Prednisolon, Prednison, Dexamethason, Betamethason, Fluprednyliden und deren Ester, z. B. Hydrocortison-21-acetat, -21-palmitat, -21-stearat, Cortison-21-acetat, -21-palmitat, -21-stearat, Prednisolon-21-acetat, -21-palmitat, -21-stearat, Prednison-21-acetat, -21-palmitat, -21-stearat, Dexamethason-21-acetat, -21-palmitat, -21-stearat, -21-phosphat, Betamethason-21-acetat, -21-palmitat, -21-stearat, -21-phosphat, Betamethason-17-valerat, Fluprednyliden-21-acetat ; Peptidhormone, wie Calcitonon ; Antibiotika, z. B. Tetracycline, Penicilline, Cephalosporine, Aminoglykoside, wie Gentamycin, Tobramycin, Amikacin,

6

Kanamycin, Neomycin, Framycetin, Streptomycin oder Netilmicin ; Chloramphenicol ; Macrolidantibiotika, wie Erythromycin und dessen Derivate, insbesondere dessen Palmitat und Stearat, oder Spiramycin ; Antimykotika und Dermatika, wie Clotrimazol, Miconazol, Dithranol, Benzoylperoxid ; Antiphlogistika, wie Indometacin, Nicotinsäuremethyl-, -benzyl- und -2-butoxyethylester. Zytostatika, wie Daunorubicin. Weiterhin kommen beispielsweise kosmetische Wirkstoffe in Betracht, z. B. Lichtschutzmittel oder Hautpflegemittel.

Liposomale Arzneimittel können, je nach Applikationsart, in die dafür geeigneten Darreichungformen gebracht werden :

— Parenteralia, insbesondere sterile Injektions- und Infusionslösungen, wobei die kolloidale Lösung der liposomalen Arzneimittel einer antimikrobiellen Behandlung unterworfen werden kann.

Für die Langzeitstabilität kann es von Vorteil sein, das liposomale Arzneimittel als Lyophilisat mit definiertem Restgehalt an wässriger Phase zu lagern und unmittelbar vor der Applikation mit der nötigen Menge an wässriger Phase zu versetzen.

— Lösungen, insbesondere Sirupe, Augen- und Nasentropfen, die neben den wie oben beschrieben herzustellenden liposomalen Arzneimitteln die für diese Arzneiformen spezifischen Zusätze enthalten können.

— Nicht dosierbare Aerosole und Dosier-Aerosole, die ausser den oben beschriebenen, liposomalen Arzneimitteln Treibmittel und Stabilisatoren enthalten können.

— Emulsionen, wobei die liposomalen Arzneimittel sich immer in der wässrigen Phase befinden, die zur parenteralen, oralen und topischen Applikation verwendet werden. Solche Emulsionen können ebenfalls zu den entsprechenden nicht dosierbaren Aerosolen und zu Dosier-Aerosolen verarbeitet werden.

— Ferner eignen sich als mögliche Darreichungsformen Hydrogele, die die liposomalen Arzneimittel beinhalten.

— Lyophilisate, aber auch anderweitig hergestellte feindisperse Mischungen aus amphiphilen, bilayerbildenden Substanzen, Lösungsvermittlern, Arzneistoffen und/oder pharmazeutischen Hilfsstoffen können in für die Applikation erforderliche Darreichungsform gebracht werden (Tabletten, Dragees, Kapseln) und nach Verabreichung durch Kontakt mit den Körperflüssigkeiten dabei direkt in liposomale Arzneimittel übergeführt werden.

## Beispiel 1 (Verdünnung)

65 mg Eierlecithin in ethanolischer Lösung werden unter Vakuum zur Trockne eingedampft und in 5 ml eines 1 mM Phosphatpuffers pH 7,3, mit NaCl auf die Ionenstärke 0,16 eingestellt, resuspendiert. Dieser Dispersion werden 51,1 mg festes Natriumcholat zugefügt und bei Raumtemperatur gerührt, wobei die erforderlichen Assoziate gebildet werden, was sich durch Klarwerden der wässrigen Phase anzeigt. Aus der für diesen Versuch eingesetzten Menge an Bilayerbildner und Lösungsvermittler resultiert ein molares Verhältnis von Bilayerbildner zu Lösungsvermittler von 0,72.

Nun wird die die Assoziate enthaltende, wässrige Phase mit Phosphatpuffer (1 mM, pH 7,3, Ionenstärke, 0,16) im Verhältnis 1 : 10 bei Raumtemperatur verdünnt, wodurch Liposomen gebildet werden, was daran zu erkennen ist, dass die Lösung Opaleszenz zeigt.

Der Restgehalt an Natriumcholat kann wenn nötig mittels Dialyse der die Liposomen enthaltenden wässrigen Phase oder durch Gelchromatographie abgetrennt werden.

Unter diesen Bedingungen hergestellte Liposomen sind unilamellar, weisen eine Grösse von 30 nm ± 2 nm im Durchmesser auf und sind in Bezug auf ihre Grössenverteilung von extremer Homogenität.

Wird ein Anfangsverhältnis von Bilayerbildner zu Lösungsvermittler von 1,15 gewählt und erfolgt die Verdünnung der die Assoziate enthaltenden Lösung im Verhältnis 1/3, resultieren ebenfalls homogene, unilamellare Liposomen mit einem Durchmesser von 44 ± 2 nm.

Weitere Präparationen von homogenen, unilamellaren Liposomen unterschiedlicher Grösse, hergestellt mit verschiedenen Bilayerbildnern und Lösungsvermittlern durch Verdünnen wie eben beschrieben, teilweise mit Einbau von lipophilen und hydrophilen Modellarzneistoffen, sind in der nachfolgenden Tabelle aufgeführt.

Tabelle

| BILAYERBILDNER<br><br>Konzentration in der wässrigen Phase bei der Assoziatbildung<br>(mg/ml) | LOESUNGSVER-MITTLER<br><br>Ausgangskonzentration<br><br><br>(mg/ml) | MOLARES VER-HAELTNIS<br>Bilayerbild-ner zu Lö-sungsver-mittler | MODELL-SUBSTANZ<br>eingesetzte Menge auf wässrige Phase bezogen<br>(mg/ml) |
|---|---|---|---|
| EYL / DSPC<br>3:1[*]<br>9,75    3,34 | Na-Cholat<br><br>9,11 | 0,8 | – |
| EYL / DSPC<br>3:1[*]<br>9,75    3,34 | $C_8$-Glycosid<br><br>24,75 | 0,2 | – |
| DSPC<br>15,81 | Na-Cholat<br>43,05 | 0,2 | – |
| EYL<br>8,0 | $C_7$-Glycosid<br>26,67 | 0,13 | Cholesterol<br>0,81 |
| EYL/Cholesterol[1]<br>5:1[*]<br>10,4    1,73 | $C_8$-Glycosid<br><br>50,0 | 0,25 | 6-Carboxyfluo-rescein<br>37,63 |

[1]) Cholesterol ist hier als Membranbestandteil zu betrachten
[2]) Abrennung der nicht gebundenen Modellsubstanz mittels Gelchromatographie oder Dialyse
[3]) Abtrennung der nicht gebundenen Modellsubstanz mittels Gelchromatographie
[*]) Molares Verhältnis

## 0 056 781

Tabelle (Fortsetzung)

| VERDUENNUNGS-VERHAELTNIS | LIPOSOMEN-GROESSE Durch-messer (nm) | EINBAURATE Modellsub-stanz in % der Aus-gangs-menge | VERSUCHSBE-DINGUNGEN |
|---|---|---|---|
| 1 : 5 | 60,5 | – | Assoziatlösung bei 56° C hergestellt. Ver-dünnung bei RT oder 56° C |
| 1 : 5 | 126 | – | Assoziatlösung bei 56° C hergestellt. Ver-dünnung bei RT oder 56° C |
| 1 : 5 | 91,4 | – | Assoziatlösung bei 60° C hergestellt. Ver-dünnung bei 60° C |
| 1 : 3 | 99 | 80 | Assoziatlösung bei RT hergestellt. Verdünnung bei RT[2] |
| 1 : 4 | 190 | 3 | Assoziatlösung bei 55° C hergestellt. Verdünnung bei 55° C[3] |

Abkürzungen
EYL = Eierlecithin
DSPC = Distearoylphosphatidylcholin
$C_8$-Glycosid = 1-0-n-octyl-β-D-glucopyranosid
$C_7$-Glycosid = 1-0-n-heptyl-β-D-glucopyranosid

Ferner wurden Versuche durchgeführt, um den Einfluss der Geschwindigkeit des Verdünnens von Assoziate enthaltenden wässrigen Phasen auf die Grösse der gebildeten Liposomen festzustellen. Dabei wurde jeweils der die Assoziate enthaltenden wässrigen Phase unter Rühren derselben eine vorbestimmte Menge zusätzlicher wässriger Phase innerhalb unterschiedlicher Zeiträume zugeführt.

In einem ersten Versuch wurden Assoziate aus Eierlecithin und Natriumcholat im molaren Verhältnis von 0,72 hergestellt. Die Konzentration des Eierlecithins betrug 26 mg/ml. Die Verdünnung erfolgte im Verhältnis 1 : 11 bei pH 7,1 und bei Raumtemperatur. Die Resultate waren die folgenden :

| Dauer des Verdünnungs-prozesses | Durchmesser der result. Liposo-men in nm | Homogenität |
|---|---|---|
| ca. 10 msec | 30 | sehr gut |
| 30 min | 47 | gut |
| 100 min | 66 | gut |
| > 3 h | nicht bestimmbar | heterogen |

Im zweiten Versuch wurden Assoziate aus Eierlecithin und Octylglucosid im molaren Verhältnis von 0,2 hergestellt. Die Konzentration des Eierlecithins betrug 20 mg/ml. Die Verdünnung erfolgte im Verhältnis 1 : 5 bei pH 7,1 und bei Raumtemperatur. Folgende Resultate wurden gefunden :

| Dauer des Verdünnungs-prozesses | Durchmesser der result. Liposo-men in nm | Homogenität |
|---|---|---|
| ca. 10 msec | 113 | gut |
| 24 min | 167 | gut |
| 50 min | 190 | gut |
| 3 h 25 min | ca. 240 | heterogen |

In einem dritten Versuch wurden Assoziate aus Distearoylphosphatidylcholin und Natriumcholat im molaren Verhältnis von 0,70 hergestellt. Die Konzentration des Distearoylphosphatidylcholins betrug 50 mg/ml. Die Verdünnung erfolgte im Verhältnis 1 : 21 bei pH 7,1 und 60 °C. Die Resultate waren die folgenden :

| Dauer des Verdünnungs-prozesses | Durchmesser der result. Liposomen in nm | Homogenität |
|---|---|---|
| ca. 10 msec | 52 | gut |
| 36 min | 100 | gut |
| 3 h 46 min | nicht bestimmbar | heterogen. |

Diese Versuche zeigen, dass man unilamellare Liposomen auch erhalten kann, wenn sich der Verdünnungsprozess über einen gewissen Zeitraum von beispielsweise bis zu etwa 3 Stunden erstreckt, und dass dabei die Grössenverteilung der Liposomen immer noch homogen ist, wenn man nur die Verdünnungsgeschwindigkeit konstant hält. Die Verdünnungsgeschwindigkeit darf jedoch in jedem Fall einen bestimmten Mindestwert nicht unterschreiten, weil sich sonst multilamellare Liposomen mit heterogener Grössenverteilung bilden.

Beispiel 2 (Dialyse)

100 mg Eierlecithin in ethanolischer Lösung werden unter Vakuum eingedampft und mit 10 ml eines 1 mM Phosphatpuffers pH 7,4, mit NaCl auf die Ionenstärke von 0,16 eingestellt und 190,4 mg 1-0-n-octyl-β-D-glucopyranosid beinhaltend, bei Raumtemperatur versetzt, wobei die erforderlichen Assoziate sofort gebildet werden.

Diese Assoziatlösung wird bei Raumtemperatur mit einer Fliessgeschwindigkeit von 0,2 ml/min im Gegenstrom, von einer Dialysierflüssigkeit (1 mM Phosphatpuffer pH 7,4, Ionenstärke 0,16) durch eine semipermeable Membran mit einer molekularen Ausschlussgrenze von 10 000 Molekulargewicht

getrennt, während 60 Minuten dialysiert. Dabei beträgt die Fliessgeschwindigkeit der Dialysierflüssigkeit 2,2 ml/min. Die nach dieser Kontaktzeit aus den Assoziaten in wässriger Lösung gebildeten unilamellaren Liposomen weisen einen Durchmesser von 120 nm auf und sind in Bezug auf ihre Grössenverteilung von extremer Homogenität, wobei der Restgehalt an Lösungsvermittler 2,6 % des anfänglichen Gehaltes beträgt. Die Bildung der Liposomen ist bereits nach 10 Minuten abgeschlossen.

Eine Anreicherung von mittels Gegenstromdialyse hergestellten liposomalen Arzneimitteln mit zusätzlichen Arzneistoffen und/oder pharmazeutischen Hilfsstoffen kann dergestalt durchgeführt werden, dass diese der Dialysierflüssigkeit in der gewünschten Menge während oder nach der Herstellung des liposomalen Arzneimittels zugesetzt werden. Dabei wird eine definierte Anreicherung der zugesetzten Stoffe in der die Liposomen enthaltenden, wässrigen Phase und bei geeigneter Wahl der Temperatur eine erhöhte Aufnahme derselben in die Liposomen ermöglicht.

## Beispiel 3 (Temperatursprung)

Distearoylphosphatidylcholin (DSPC) wird wie beschrieben unter Zusatz von Natriumcholat im molaren Verhältnis von 0,2 in einen Lipidfilm übergeführt, und bei 60 °C werden durch Zusatz von 10 mM Phosphatpuffer pH 7,1 die Assoziate gebildet. Die Lipidkonzentration der klaren Assoziatlösung beträgt 5 mg/ml. Wird diese Assoziatlösung langsam auf Raumtemperatur abkühlen gelassen, so entstehen innerhalb von 4 Stunden homogene, unilamellare Liposomen mit einem mittleren Durchmesser von 260 nm. Erfolgt dieser Temperatursprung über einen grösseren Bereich, z. B. von 60 °C auf 4 °C, und damit entsprechend rascher, so resultieren kleinere homogene Liposomen mit einem mittleren Durchmesser von 75 nm.

## Beispiel 4 (pH-Sprung)

Phosphatidylcholin aus Eigelb wird unter Zusatz von Natriumcholat im molaren Verhältnis von 0,72 wie beschrieben in einen Lipidfilm übergeführt, und bei Raumtemperatur werden durch Zusatz von 10 mM Phosphatpuffer, pH 7,1 die Assoziate gebildet. Die Lipidkonzentration der klaren Assoziatlösung beträgt 26 mg/ml. Wird durch schnellen Zusatz von 0,1 N Salzsäure des pH dieser klaren Assoziatlösung auf pH 3 herabgesetzt, so wird der Lösungsvermittler ausgefällt, wobei sich im Ueberstand homogene Liposomen mit einem mittleren Durchmesser von 30 nm bilden.

## Beispiel 5 (Erhöhung der Bilayerbildnerkonzentration)

100 mg Phosphatidylcholin aus Eigelb werden zusammen mit 60 mg Natriumcholat, woraus ein molares Verhältnis von Lecithin zu Natriumcholat von 0,8 resultiert, wie beschrieben in einen Lipidfilm überführt, und bei Raumtemperatur werden durch Zusatz von 5 ml 10 mM Phosphatpuffer, pH 7,1, die Assoziate gebildet. In einem zweiten Gefäss werden 49,75 mg Phosphatidylcholin lyophilisiert. Diesem hochdispersen Lyophilisat wird die oben beschriebene Assoziatlösung (5 ml) unter Rühren augenblicklich zugefügt, wobei sich durch Aenderung des molaren Verhältnisses von ursprünglich 0,8 auf 1,2 zwischen Bilayerbildner und Lösungsvermittler spontan Liposomen mit einem mittleren Durchmesser von 70 nm bilden.

In den beschriebenen Ausführungsformen wurde zumeist davon ausgegangen, dass die anfänglich eingesetzte Lösungsvermittlermenge so gross ist, dass die gesamte vorliegende Menge an bilayerbildender Substanz solubilisiert ist (d. h. in Form von Assoziaten vorliegt), und dass dann der Lösungsvermittlergehalt so stark herabgesetzt wird, dass sich aus allen Assoziaten in der wässrigen Phase Liposomen bilden. Beides ist jedoch nicht in allen Fällen erforderlich. Man kann einerseits auch von einer wässrigen Phase ausgehen, die neben Assoziaten auch noch dispergierte bilayerbildende Substanz enthält oder mit solcher (beispielsweise in Filmform) in Berührung steht. Und man kann andererseits auch den Lösungsvermittlergehalt nur relativ wenig herabsetzen, so dass sich nur ein Teil der Assoziate in der wässrigen Phase zu Liposomen vereinigen, während andere Assoziate als solche bestehen bleiben. Man kann sogar auch beide Möglichkeiten zusammen anwenden und dann ein Produkt erhalten, das dispergierte bilayerbildende Substanz, Assoziate und Liposomen enthält.

## Anwendungsbeipiel : Hydrogel

(a) Analog Beispiel 1 löst man 320 mg Eierlecithin, 80 mg Cholesterin und 40 mg Betamethason-17-valerat in Ethanol, dampft ein, resuspendiert in 20 ml Phosphatpuffer, versetzt mit 400 mg Na-Cholat und verfährt weiter analog Beispiel 1.

(b) In 75 ml Wasser löst man 0,2 g Kaliumsorbat, 0,224 g Dinatriumhydrogenphosphat-12-hydrat und 0,64 g Kaliumdihydrogenphosphat. Unter leichtem Erwärmen und kräftigem Rühren werden in der erhaltenen Lösung 2 g Hydroxyethylcellulose gelöst. Man lässt noch 0,6 Stunden durchquellen, rührt zuerst 2 g Glycerin und dann die nach (a) erhaltene Liposomendispersion ein und fügt Wasser ad 100 ml hinzu.

Das erhaltene Hydrogel enthält 0,04 % Wirkstoff und zeigt einen pH-Wert von 5,8-6,3.

**Patentansprüche**

1. Verfahren zur Herstellung von liposomalen Arzneimitteln, wobei in wässriger Phase aus wenigstens einer amphiphilen, bilayerbildenden Substanz und einem Lösungsvermittler Assoziate gebildet werden, dadurch gekennzeichnet, dass in der die Assoziate enthaltenden wässrigen Phase durch Verdünnung derselben die Konzentration an freiem gelöstem Lösungsvermittler erniedrigt wird und damit die Gleichgewichtsbedingungen für das molare Verhältnis zwischen bilayerbildender Substanz und Lösungsvermittler in den Assoziaten im Sinne einer Erhöhung dieses Verhältnisses mit einer Geschwindigkeit geändert werden, die so hoch ist, dass sich Assoziate zu Liposomen mit definierter Anzahl Doppelschichten vereinigen, und praktisch konstant ist, so dass die gebildeten Liposomen eine definierte, praktisch homogene Grösse haben.

2. Verfahren nach Anspruch 1, dadurch gekennzeichnet, dass die totale Lösungsvermittlerkonzentration in der wässrigen Phase durch Verdünnung mit zusätzlicher wässriger Phase auf höchstens 50 %, vorzugsweise höchstens 25 %, der Ausgangskonzentration herabgesetzt wird.

3. Verfahren zur Herstellung von liposomalen Arzneimitteln, wobei in wässriger Phase aus wenigstens einer amphiphilen, bilayerbildenden Substanz und einem Lösungsvermittler Assoziate gebildet werden, dadurch gekennzeichnet, dass in der die Assoziate enthaltenden wässrigen Phase Lösungsvermittler durch chemische und/oder physikalisch-chemische Reaktion inaktiviert wird, um die Konzentration an aktivem Lösungsvermittler in der wässrigen Phase auf höchstens 50 %, vorzugsweise höchstens 25 %, der Ausgangskonzentration herabzusetzen, und damit in der wässrigen Phase die Gleichgewichtsbedingungen für das molare Verhältnis zwischen bilayerbildender Substanz und Lösungsvermittler in den Assoziaten im Sinne einer Erhöhung dieses Verhältnisses mit einer Geschwindigkeit geändert werden, die so hoch ist, dass sich Assoziate zu Liposomen mit definierter Anzahl Doppelschichten vereinigen, und praktisch konstant ist, so dass die gebildeten Liposomen eine definierte, praktisch homogene Grösse haben.

4. Verfahren zur Herstellung von liposomalen Arzneimitteln, wobei in wässriger Phase aus wenigstens einer amphiphilen, bilayerbildenden Substanz und einem Lösungsvermittler Assoziate gebildet werden, dadurch gekennzeichnet, dass in der die Assoziate enthaltenden wässrigen Phase die Konzentration an freiem gelöstem Lösungsvermittler mittels Gegenstromdialyse erniedrigt wird, indem in mindestens zwei jeweils durch eine semipermeable Membran getrennten Kanälen die die Assoziate enthaltende wässrige Phase und eine Dialysierflüssigkeit, durch die semipermeable Membran voneinander getrennt, strömen gelassen werden, und damit in der wässrigen Phase die Gleichgewichtsbedingungen für das molare Verhältnis zwischen bilayerbildender Substanz und Lösungsvermittler in den Assoziaten im Sinne einer Erhöhung dieses Verhältnisses mit einer Geschwindigkeit geändert werden, die so hoch ist, dass sich Assoziate zu Liposomen mit definierter Anzahl Doppelschichten vereinigen, und praktisch konstant ist, so dass die gebildeten Liposomen eine definierte, praktisch homogene Grösse haben.

5. Verfahren nach Anspruch 4, dadurch gekennzeichnet, dass die Fliessgeschwindigkeit der zu dialysierenden wässrigen Phase derart gewählt wird, dass nach Passieren der Kanallänge mindestens 50 %, vorzugsweise mindestens 75 %, der dialysierbaren Stoffe aus der zu dialysierenden wässrigen Phase entfernt sind.

6. Verfahren nach Anspruch 4 oder 5, dadurch gekennzeichnet, dass der Dialysierflüssigkeit wenigstens ein dialysierbarer Arzneistoff und/oder ein pharmazeutischer Hilfsstoff zugesetzt wird, so dass durch Diffusion durch die semipermeable Membran das liposomale Arzneimittel mit Arzneistoff und/oder pharmazeutischem Hilfsstoff angereichert wird.

7. Verfahren zur Herstellung von liposomalen Arzneimitteln, wobei in wässriger Phase aus wenigstens einer amphiphilen, bilayerbildenden Substanz und einem Lösungsvermittler Assoziate gebildet werden, dadurch gekennzeichnet, dass in der die Assoziate enthaltenden wässrigen Phase durch Zugeben von zusätzlicher bilyerbildender Substanz in fester oder in wässriger Phase gelöster und/oder dispergierter Form die Konzentration an freiem gelöstem Lösungsvermittler erniedrigt wird und damit die Gleichgewichtsbedingungen für das molare Verhältnis zwischen bilayerbildender Substanz und Lösungsvermittler in den Assoziaten im Sinne einer Erhöhung dieses Verhältnisses mit einer Geschwindigkeit geändert werden, die so hoch ist, dass sich Assoziate zu Liposomen mit definierter Anzahl Doppelschichten vereinigen, und praktisch konstant ist, so dass die gebildeten Liposomen eine definierte, praktisch homogene Grösse haben.

8. Verfahren nach einem der Ansprüche 1 bis 7, dadurch gekennzeichnet, dass die Liposomen oder liposomalen Arzneimittel mit wenigstens einem Arzneistoff und/oder einem pharmazeutischen Hilfsstoff unmittelbar bei oder oberhalb der Uebergangstemperatur inkubiert werden und der Arzneistoff und/oder der pharmazeutische Hilfsstoff damit in die Liposomen aufgenommen bzw. eingebaut wird.

**Claims**

1. Process for the preparation of liposomal medicaments, associates of at least one amphiphilic bilayer-forming substance and a solubilising agent being formed in an aqueous phase, characterised in that, in the aqueous phase containing the associates, the concentration of free dissolved solubilising agent is lowered by diluting the aqueous phase so as to change the equilibrium conditions for the molar ratio of the bilayer-forming substance and the solubilising agent in the associates in the sense of an increase in this ratio, at a rate which is high enough for associates to combine to form liposomes with a defined number of double layers, and which is virtually constant, so that the liposomes formed have a defined, virtually homogeneous size.

2. Process according to Claim 1, characterised in that the total concentration of solubilising agent in the aqueous phase is reduced to at most 50 %, preferably at most 25 %, of the starting concentration by dilution with additional aqueous phase.

3. Process for the preparation of liposomal medicaments, associates of at least one amphiphilic bilayer-forming substance and a solubilising agent being formed in an aqueous phase, characterised in that, in the aqueous phase containing the associates, solubilising agent is inactivated by a chemical and/or physicochemical reaction in order to reduce the concentration of active solubilising agent in the aqueous phase to at most 50 %, preferably at most 25 %, of the starting concentration, so as to change, in the aqueous phase, the equilibrium conditions for the molar ratio of the bilayer-forming substance and the solubilising agent in the associates in the sense of an increase in this ratio, at a rate which is high enough for associates to combine to form liposomes with a defined number of double layers, and which is virtually constant, so that the liposomes formed have a defined, virtually homogeneous size.

4. Process for the preparation of liposomal medicaments, associates of at least one amphiphilic bilayer-forming substance and a solubilising agent being formed in an aqueous phase, characterised in that, in the aqueous phase containing the associates, the concentration of free dissolved solubilising agent is lowered by countercurrent dialysis, by allowing the associate-containing aqueous phase and a dialysing liquid to flow in at least two channels in each case separated by a semipermeable membrane, the liquids being separated from one another by the semipermeable membrane, so as to change, in the aqueous phase, the equilibrium conditions for the molar ratio of the bilayer-forming substance and the solubilising agent in the associates in the sense of an increase in this ratio, at a rate which is high enough for associates to combine to form liposomes with a defined number of double layers, and which is virtually constant, so that the liposomes formed have a defined, virtually homogeneous size.

5. Process according to Claim 4, characterised in that the flow rate of the aqueous phase to be dialysed is chosen such that after the aqueous phase has passed along the length of the channel, at least 50 %, preferably at least 75 %, of the dialysable substances have been removed from the aqueous phase to be dialysed.

6. Process according to Claim 4 or 5, characterised in that at least one dialysable pharmaceutical substance and/or a pharmaceutical auxiliary is added to the dialysing liquid so that the liposomal medicament becomes enriched with the pharmaceutical substance and/or pharmaceutical auxiliary as a result of diffusion through the semipermeable membrane.

7. Process for the preparation of liposomal medicaments, associates of at least one amphiphilic bilayer-forming substance and a solubilising agent being formed in an aqueous phase, characterised in that, in the aqueous phase containing the associates, the concentration of free dissolved solubilising agent is lowered by adding additional bilayer-forming substance in solid form or as a solution and/or dispersion in aqueous phase so as to change the equilibrium conditions for the molar ratio of the bilayer-forming substance and the solubilising agent in the associates in the sense of an increase in this ratio, at a rate which is high enough for associates to combine to form liposomes with a defined mumber of double layers, and which is virtually constant, so that the liposomes formed have a defined, virtually homogeneous size.

8. Process according to one of Claims 1 to 7, characterised in that the liposomes or liposomal medicaments are incubated with at least one pharmaceutical substance and/or pharmaceutical auxiliary immediately at or above the transition temperature and the pharmaceutical substance and/or the pharmaceutical auxiliary is thus absorbed or incorporated into the liposomes.

**Revendications**

1. Procédé pour préparer des médicaments liposomaux, dans lequel on forme en phase aqueuse des produits d'association à partir d'au moins une substance amphiphile génératrice de couches doubles et d'un tiers-solvant, procédé caractérisé en ce que, dans la phase aqueuse contenant les produits d'association, on diminue, par dilution de cette phase, la concentration en tiers-solvant libre dissous et l'on modifie ainsi les conditions d'équilibre pour le rapport molaire entre la substance génératrice de couches doubles et le tiers-solvant dans les produits d'association, dans le sens d'une élévation de ce rapport, en opérant à une vitesse suffisamment élevée pour que des produits d'association se regroupent en des liposomes ayant un nombre bien défini de doubles couches, cette vitesse étant pratiquement constante de manière que les liposomes formés possèdent une grosseur bien définie et pratiquement homogène.

2. Procédé selon la revendication 1, caractérisé en ce que, par dilution avec un supplément de phase aqueuse, on diminue la concentration totale en tiers-solvant dans la phase aqueuse jusqu'à un maximum de 50 %, de préférence un maximum de 25 %, de la concentration initiale.

3. Procédé pour préparer des médicaments liposomaux, dans lequel on forme en phase aqueuse des produits d'association à partir d'au moins une substance amphiphile génératrice de couches doubles et d'un tiers-solvant, procédé caractérisé en ce qu'on inactive du tiers-solvant dans la phase aqueuse contenant les produits d'association par réaction chimique et/ou physico-chimique afin de diminuer la concentration en tiers-solvant actif dans la phase aqueuse pour la faire parvenir à une valeur maximale de 50 %, de préférence au maximum de 25 %, de la concentration initiale, et l'on modifie ainsi dans la phase aqueuse les conditions d'équilibre pour le rapport molaire entre la substance génératrice de couches doubles et le tiers-solvant dans les produits d'association, dans le sens d'une élévation de ce rapport, en opérant à une vitesse suffisamment élevée pour que des produits d'association se regroupent en des liposomes ayant un nombre bien défini de doubles couches, cette vitesse étant pratiquement constante de manière que les liposomes formés possèdent une grosseur bien définie et pratiquement homogène.

4. Procédé pour préparer des médicaments liposomaux, dans lequel on forme en phase aqueuse des produits d'association à partir d'au moins une substance amphiphile génératrice de couches doubles et d'un tiers-solvant, procédé caractérisé en ce que, dans la phase aqueuse contenant les produits d'association, on diminue, par dialyse à contrecourant, la concentration en tiers-solvant libre dissous, en faisant couler en au moins deux canaux, séparés chaque fois par une membrane semi-perméable, la phase aqueuse contenant les produits d'association et un liquide de dialyse, séparés l'un de l'autre par la membrane semi-perméable, et l'on modifie ainsi dans la phase aqueuse les conditions d'équilibre pour le rapport molaire entre la substance génératrice de couches doubles et le tiers-solvant dans les produits d'association, dans le sens d'une élévation de ce rapport, à une vitesse suffisamment élevée pour que des produits d'association se regroupent en des liposomes ayant un nombre bien défini de doubles couches, cette vitesse étant pratiquement constante de manière que les liposomes formés possèdent une grosseur bien définie et pratiquement homogène.

5. Procédé selon la revendication 4, caractérisé en ce qu'on choisit la vitesse d'écoulement de la phase aqueuse à dialyser de manière qu'après avoir emprunté la longueur des canaux, la phase aqueuse à dialyser ait subi un enlèvement d'au moins 50 %, avantageusement d'au moins 75 %, des substances dialysables.

6. Procédé selon la revendication 4 ou 5, caractérisé en ce qu'on ajoute au liquide de dialyse au moins une substance médicinale dialysable et/ou un adjuvant pharmaceutique dialysable, de sorte que, par diffusion à travers la membrane semi-perméable, le médicament liposomal s'enrichisse en substance médicinale et/ou en adjuvant pharmaceutique.

7. Procédé pour préparer des médicaments liposomaux, dans lequel on forme en phase aqueuse des produits d'association à partir d'au moins une substance amphiphile génératrice de couches doubles et d'un tiers-solvant, procédé caractérisé en ce que, par addition d'un supplément de substance génératrice de couches doubles, sous forme solide ou dissoute et/ou dispersée dans la phase aqueuse, on diminue dans la phase aqueuse contenant les produits d'association, la concentration en tiers-solvant libre dissous et l'on modifie ainsi les conditions d'équilibre pour le rapport molaire entre la substance génératrice de couches doubles et le tiers-solvant dans les produits d'association, dans le sens d'une élévation de ce rapport, en opérant à une vitesse suffisamment élevée pour que des produits d'association se regroupent en des liposomes ayant un nombre bien défini de doubles couches, cette vitesse étant pratiquement constante de manière que les liposomes formés possèdent une grosseur bien définie et pratiquement homogène.

8. Procédé selon l'une des revendications 1 à 7, caractérisé en ce qu'on fait incuber, immédiatement à la température de transformation ou au-dessus de cette température, les liposomes ou médicaments liposomaux avec au moins une substance médicinale et/ou un adjuvant pharmaceutique et l'on provoque ainsi la pénétration de la substance médicinale et/ou de l'adjuvant pharmaceutique dans les liposomes ou son ou leur incorporation aux liposomes.